# EUROPÄISCHE PATENTANMELDUNG

(11) **EP 0 825 723 A1**
(43) Veröffentlichungstag der Anmeldung: **25.02.1998**
(21) Anmeldenummer: 96810548.6
(22) Anmeldetag: 16.08.1996
(51) Int. Cl.: H04B 1/12, A61B 5/0428, A61B 5/04, G06F 19/00

(54) **Verfahren zur Abschwächung einer Störung bei der Auswertung eines bioelektrischen Messsignals**

(71) Anmelder: Dr.-Ing. P. Osypka GmbH, 79639 Grenzach-Wyhlen (DE)
(72) Erfinder: Geistert, Wolfgang, Dr., 79618 Rheinfelden-Herten (DE)
(74) Vertreter: Heubeck, Bernhard

(57) **Zusammenfassung**

Das Verfahren betrifft die Abschwächung einer Störung bei der Auswertung eines bioelektrischen Messsignals, das sich aus einem Nutzsignal und einer Störkomponente zusammensetzt. Es wird von folgenden Voraussetzungen ausgegangen: a) Der Störkomponente lassen sich wiederkehrende Störsignale einer Störquelle zuordnen. b) Der Unterschied zwischen dem Nutzsignal und dem Störsignal ist durch mindestens einen Parameter charakterisierbar. c) Aufgrund dieses Parameters oder einer Kombination dieser Parameter ist das Störsignal identifizierbar. In einer Logikschaltung werden mit einem Erkennungsalgorithmus Messwerte bearbeitet, wobei unter den genannten Voraussetzungen und mittels des Parameters oder der Parameterkombination aus den Messwerten ein angenähert richtiges Störsignal extrahiert wird. Das extrahierte Störsignal wird schliesslich von dem Messsignal subtrahiert.

## Beschreibung

Die Erfindung betrifft ein Verfahren zur Abschwächung einer Störung bei der Auswertung eines bioelektrischen Messsignals gemäss Oberbegriff von Anspruch 1 sowie die Anwendung des Verfahrens bei EKG-Signalen.

Bei der Diagnose der Herztätigkeit von Patienten mittels Elektrokardiographen sind die EKG-Messsignale in der Regel von Störungen überlagert, welche die Erkennung von Details im Signal erschweren oder unmöglich machen. Störquelle ist das Stromversorgungsnetz ("Netzbrumm"), insbesondere Netzkabel sowie Transformatoren und Drosselspulen, die in fast allen elektrischen Geräten enthalten sind. Die erwähnten, an das Netz angeschlossenen Teile haben die Wirkung von Sendeantennen; der Körper des Patienten und die Messgeräte wirken als Empfänger. Auch andere bioelektrische Signale wie z.B. Encephalographen-Messsignale sind Störungen unterworfen, die eine Diagnose erschweren.

Der grösste Teil des Spektralbereiches der EKG-Signale liegt im Bereich von 0.1 - 500 Hz. Somit liegen die Grundfrequenz der "Netzbrumm"-Störung (Netzfrequenz 50 oder 60 Hz) und deren ersten Oberwellen mitten in diesem Spektralbereich.

Es ist bekannt, die "Netzbrumm"-Störungen mit Hilfe von Filtern (Notch-Filter) zu eliminieren. Diese Massnahme führt allerdings zu erheblichen Verfälschungen des Messsignals. Denn durch die Filter entstehen im Spektrum des Signals bei der Frequenz des Netzes und/oder den entsprechenden Oberschwingungs-Frequenzen Lücken, die das Signal entstellen. Ferner können sich bei der Verwendung von Notch-Filtern zusätzliche Störungen ergeben, die durch Schwingphänomene der Filter verursacht werden.

Es ist daher Aufgabe der Erfindung, ein Verfahren zur Abschwächung einer Störung bei der Auswertung eines bioelektrischen Messsignals zu schaffen, bei dem das Verfahren selbst keine wesentliche Verfälschung des Signals verursacht. Diese Aufgabe wird mit dem in Anspruch 1 definierten Verfahren gelöst.

Die abhängigen Ansprüche 2 bis 10 beziehen sich auf bevorzugte Ausführungsformen des erfindungsgemässen Verfahrens. Gegenstand des Anspruchs 11 ist die Anwendung des Verfahrens auf EKG-Signale.

Die der Erfindung zugrunde liegende Idee beruht auf der Beobachtung, dass die den EKG-Signalen überlagerten Netz-Störungen hinsichtlich Frequenz, Amplitude und Kurvenform in der Regel über einen längeren Zeitraum regelmässiger Natur sind. Erleichtert wird das erfindungsgemässe Verfahren dadurch, dass die Messsignale, insbesondere EKG-Signale, meist längere isopotentiale Teilstücke aufweisen, innerhalb derer die Signale nur wenig von einer Nulllinie abweichen.

Bei einer bevorzugten Ausführungsform der Erfindung wird das durch das gestörte Messsignal gebildete Eingangssignal in einem Analog-Digital-Wandler in digitale Daten umgesetzt und diese Daten in einen Mikroprozessor eingegeben. Ausserdem wird aus der Versorgungsspannung ein zur Netzfrequenz phasensynchrones Triggersignal erzeugt, das ebenfalls dem Mikroprozessor zur Verfügung gestellt wird. Der Mikroprozessor extrahiert mittels eines Erkennungsalgorithmus ein Störsignal und subtrahiert dieses vom Messignal, so dass ein Ausgangssignal entsteht, dem die Störkomponente ganz oder teilweise fehlt. Der Erkennungsalgorithmus benutzt die Frequenz (oder die Periodendauer), die Periodik und die Amplitudenstabilität des Störsignals, um das Störsignal vom Nutzsignal zu unterscheiden.

Der Erkennungsalgorithmus zerlegt das Eingangssignal in Teilstücke, deren Länge jeweils durch den Abstand zwischen zwei netzsynchronen Triggersignalen, d.h. durch die Periodendauer der Störquelle, gegeben ist. Mit Vorteil wird über mehrere Teilstücke gemittelt, indem sozusagen eine Periode über die andere gelegt wird. Dabei gleichen sich Schwankungen der Störung aus.

Um sicherzustellen, dass es sich bei den erfassten Teilstücken tatsächlich um die Störung handelt, wird ein Ähnlichkeitsvergleich durchgeführt: es werden die Amplituden der erfassten, digitalisierten Teilstücke miteinander verglichen. Nur wenn die Differenz einen vorgegebenen Grenzwert nicht überschreitet, werden die erfassten Teilstücke als Störung akzeptiert. Dieses einfache Vorgehen ist bei der Auswertung von EKG-Signalen möglich, da diese isopotentiale Bereiche aufweisen, in denen keine Vermischung von Störung und EKG-Deflektion auftritt. In den Teilstücken, die in den isopotentialen Bereichen auftreten, akzeptiert der Algorithmus die Störungen; während den EKG-Deflektionen verwirft er die erfassten Teilstücke.

In einer anderen Auslegungsform der Erfindung wird die Kurvenformstabilität für die Unterscheidung von Nutzsignal und Störsignal herangezogen. Dazu werden die erfassten, digitalisierten Teilstücke Punkt für Punkt miteinander verglichen. Wenn eine Ähnlichkeit vorliegt, werden die erfassten Teilstücke als Störung akzeptiert.

Der Grenzwert für den Ähnlichkeitsvergleich wird mit Vorteil derart angepasst, dass er automatisch erhöht wird, wenn über einen vorgegebenen Zeitraum keine Teilstücke als Störung akzeptiert worden sind, und dass er nach erfolgreichem Ähnlichkeitsvergleich wieder abgesenkt wird. Dieses Vorgehen erlaubt es, den Algorithmus optimal auf eine mehr oder weniger variierende Amplitude oder Kurvenform der Störung einzustellen.

Es ist vorteilhaft, wenn das Störsignal in einem Zwischenspeicher abgelegt wird. Das erkannte Störsignal muss aber nicht notwendigerweise abgespeichert werden; es könnte auch vom Erkennungsalgorithmus direkt für die Behandlung des Eingangssignals verwendet werden.

Der Erkennungsalgorithmus arbeitet mit Vorteil ständig, so dass die in einem Speicher abgelegte Störung laufend aktualisiert wird. Dadurch ist das Verfahren in der Lage, sich an wechselnde Störamplituden und Kurvenformen anzupassen. Der Erkennungsalgorithmus könnte auch einmalig, auf Aufforderung oder automatisch nach vorgegebenen Zeitintervallen gestartet werden.

Die Erkennung der Störsignale, gegebenenfalls deren Speicherung sowie die Subtraktion erfolgen mit Vorteil digital. Alle oder einzelne der Schritte des erfindungsgemässen Verfahren können aber auch auf analogem Wege ausgeführt werden.

Damit das erfindungsgemässe Verfahren erfolgreich durchführbar ist, muss es grundsätzlich möglich sein, ein Störsignal von einem Nutzsignal oder Originalsignal, dem die Störung überlagert ist, zu unterscheiden. Bei EKG-Signalen ist eine Unterscheidung möglich, da das Nutzsignal nicht die gleiche Periodendauer wie die Störung hat und diese ausserdem eine über längere Zeiträume einigermassen gleichbleibende Amplitude und Kurvenform aufweist.

Das anhand einer besonderen Ausführungsform der Erfindung beschriebene Verfahren lässt sich gemäss dem Anspruch 1 verallgemeinern: Das Verfahren betrifft die Abschwächung einer Störung bei der Auswertung eines bioelektrischen Messsignals, das sich aus einem Nutzsignal und einer Störkomponente zusammensetzt. Es wird von folgenden Voraussetzungen ausgegangen: a) Der Störkomponente lassen sich wiederkehrende Störsignale einer Störquelle zuordnen. b) Der Unterschied zwischen dem Nutzsignal und dem Störsignal ist durch mindestens einen Parameter charakterisierbar. c) Aufgrund dieses Parameters oder einer Kombination dieser Parameter ist das Störsignal identifizierbar. In einer Logikschaltung werden mit einem Erkennungsalgorithmus Messwerte bearbeitet, wobei unter den genannten Voraussetzungen und mittels des Parameters oder der Parameterkombination aus den Messwerten ein angenähert richtiges Störsignal extrahiert wird. Das extrahierte Störsignal wird schliesslich von dem Messsignal subtrahiert.

Der Parameter, der den Unterschied zwischen Störsignal und Nutzsignal charakterisiert, oder die entsprechende Parameterkombination sind durch folgende Alternativen oder Kombinationen dieser Alternativen gegeben:
- eine Frequenz des Störsignals oder des Nutzsignals,
- eine Periodik, insbesondere die Periodik eines periodisch wiederkehrenden Störsignals,
- eine Amplitude,
- eine Flankensteilheit,
- eine Kurvenform, und/oder
- die Stabilität eines der genannten Parameter.

Der Erkennungsalgorithmus kann ständig ausgeführt oder nur zeitweise ausgeführt werden. Das extrahierte Störsignal kann in einem Zwischenspeicher abgelegt werden. Die Logikschaltung kann einen Mikroprozessor umfassen.

Falls die Störsignale periodisch wiederkehren, so kann das extrahierte Störsignal durch Mittelung über mehrere Perioden gewonnen werden.

Nachfolgend wird die Erfindung anhand der Zeichnungen erläutert. Es zeigen:
- Fig. 1: ein EKG-Messsignal, dem eine "Netzbrumm"-Störung überlagert ist,
- Fig. 2: eine Prinzipdarstellung der erfindungsgemässen Störeliminierung und
- Fig. 3: ein EKG-Signal nach der Störeliminierung.

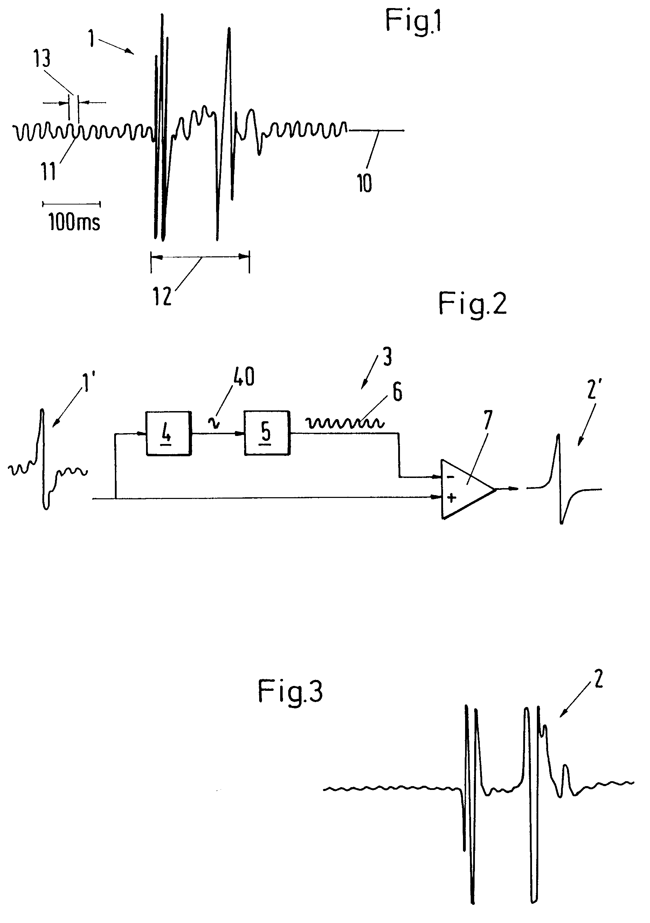

Das in Fig.1 dargestellte EKG-Messsignal 1 erstreckt sich über längere isopotentiale Teilstücke 11, in denen das EKG-Signal nur wenig von einer Nulllinie 10 abweicht, und über kürzere Zeitintervalle 12, in denen die für die Diagnose relevante Information, nämlich das Nutzsignal, erscheint. Innerhalb den isopotentialen Teilstücken 11 erkennt man deutlich die durch das Stromversorgungsnetz verursachte Störung. Diese Störung lässt sich in Signalperioden aufteilen, deren zeitlichen Intervalle 13 der Frequenz der Störquelle entsprechen und im vorliegenden Beispiel 20 ms lang sind.

Mit der in Fig.2 schematisch dargestellten Einrichtung 3 wird das erfindungsgemässe Verfahren durchgeführt. In dem ersten Block 4 der Einrichtung 3 arbeitet der Erkennungsalgorithmus. Dieser extrahiert aus dem Eingangssignal 1' die diesem überlagerte Störung in Form einer gemittelten Signalperiode 40. Im nachfolgenden Block 5 wird die Signalperiode 40 abgespeichert. Eine periodische Folge 6 der Signalperioden 40 wird schliesslich im Block 7 von dem Eingangssignal 1' abgezogen. Dem entstehenden Ausgangssignal 2' fehlt eine periodische Komponente mit der Frequenz der Störquelle ganz (wie in Fig.2 dargestellt) oder zumindest weitgehend.

Wird ein EKG-Signal 1, wie es in der Fig.1 gezeigt ist, in die Einrichtung 3 der Fig.2 eingegeben, so resultiert ein Signal 2 mit abgeschwächter Störungskomponente. Ein Beispiel für ein derartiges Signal 2 ist in Fig.3 zu sehen.

## Patentansprüche

1. Verfahren zur Abschwächung einer Störung bei der Auswertung eines bioelektrischen Messsignals (1'), das sich aus einem Nutzsignal (2') und einer Störkomponente (6) zusammensetzt,
dadurch gekennzeichnet, dass der Störkomponente wiederkehrende Störsignale (40) einer Störquelle zuordenbar sind, dass der Unterschied zwischen dem Nutzsignal und dem Störsignal durch mindestens einen Parameter charakterisierbar ist, dass aufgrund dieses Parameters oder einer Kombination dieser Parameter das Störsignal identifizierbar ist, dass in einer Logikschaltung mit einem Erkennungsalgorithmus Messwerte bearbeitet werden, wobei aus den Messwerten mittels des Parameters oder der Parameterkombination ein angenähert richtiges Störsignal extrahiert wird, und dass das extrahierte Störsignal schliesslich von dem Messsignal subtrahiert wird.

2. Verfahren nach Anspruch 1, dadurch gekennzeichnet, dass der charakterisierende Parameter oder die Parameterkombination durch folgende Alternativen beziehungsweise Kombinationen dieser Alternativen gegeben sind:
- eine Frequenz des Störsignals oder Nutzsignals,
- eine Periodik, insbesondere die Periodik eines periodisch wiederkehrenden Störsignals,
- eine Amplitude,
- eine Flankensteilheit,
- eine Kurvenform, und/oder
- die Stabilität eines der genannten Parameter.

3. Verfahren nach Anspruch 1 oder 2, dadurch gekennzeichnet, dass der Erkennungsalgorithmus ständig arbeitet oder dass er nur zeitweise ausgeführt wird und insbesondere einmalig, auf Aufforderung oder automatisch nach vorgegebenen Zeitintervallen gestartet wird.

4. Verfahren nach einem der Ansprüche 1 bis 3, dadurch gekennzeichnet, dass das Störsignal in einem Zwischenspeicher abgelegt wird, wobei insbesondere die Logikschaltung das abgespeicherte Signal ständig aktualisiert.

5. Verfahren nach einem der Ansprüche 1 bis 4, dadurch gekennzeichnet, dass die Logikschaltung einen Mikroprozessor umfasst.

6. Verfahren nach einem der Ansprüche 1 bis 5, dadurch gekennzeichnet, dass das Messsignal digitalisiert wird und die so erzeugten Daten in die Logikschaltung eingegeben werden.

7. Verfahren nach einem der Ansprüche 1 bis 6, dadurch gekennzeichnet, dass die Störsignale periodisch wiederkehren und das extrahierte Störsignal durch Mittelung über mehrere Perioden gewonnen wird.

8. Verfahren nach Anspruch 7, dadurch gekennzeichnet, dass der Erkennungsalgorithmus das Störsignal bei der Bearbeitung nur akzeptiert, wenn es mit dem bereits extrahierten Störsignal eine Ähnlichkeit aufweist und sich bei dem Ähnlichkeitsvergleich keine über einen vorgegebenen Grenzwert gehende Abweichung ergibt.

9. Verfahren nach Anspruch 8, dadurch gekennzeichnet, dass der Grenzwert für den Ähnlichkeitsvergleich derart adaptiert wird, dass bei häufiger Nicht-Akzeptanz des Störsignals der Grenzwert um vorgegebene Beträge angehoben und später wieder abgesenkt wird.

10. Verfahren nach einem der Ansprüche 1 bis 9, dadurch gekennzeichnet, dass die Störquelle durch das Stromversorgungsnetz gegeben ist, wobei Frequenz, Periodik sowie Amplituden- und/oder Kurvenformstabilität des Störsignals charakterisierende Parameter darstellen.

11. Anwendung des Verfahrens gemäss einem der Ansprüche 1 bis 10 zur Bearbeitung von EKG-Messsignalen.
